# EUROPÄISCHE PATENTSCHRIFT

(11) **EP 1 839 692 B1**
(45) Veröffentlichungstag und Bekanntmachung des Hinweises auf die Patenterteilung: **28.11.2007**
(21) Anmeldenummer: 06006679.2
(22) Anmeldetag: 30.03.2006
(51) Int. Cl.: A61M 5/142

(54) **Infusionssystem mit einer Infusionseinheit und einer Fernsteuereinheit**
Infusion system comprising an infusion unit and a remote control unit
Système de perfusion comprenant un dispositif de perfusion et un dispositif de télécommande

(43) Veröffentlichungstag der Anmeldung: 03.10.2007
(73) Patentinhaber: Roche Diagnostics GmbH, 68305 Mannheim (DE); F.HOFFMANN-LA ROCHE AG, 4070 Basel (CH)
(72) Erfinder: Rasch-Menges, Jürgen, 68723 Schwetzingen (DE); Haar, Hans-Peter, 69168 Wiesloch (DE); Poredda, Andreas, 68259 Mannheim (DE); Haueter, Ulrich, 3506 Grosshöchstetten (CH)
(74) Vertreter: Pfeifer, Hans-Peter

(56) Entgegenhaltungen:
- EP-B- 1 109 586
- WO-A-01/70307
- DE-A1- 10 223 192
- US-A- 5 573 506
- US-A1- 2005 137 530

## Beschreibung

Die vorliegende Erfindung betrifft ein Infusionssystem mit einer Infusionseinheit und einer Fernsteuereinheit, wobei die Infusionseinheit zur Anordnung außerhalb des Körpers und zur Infusion einer Flüssigkeit in den Körper ausgebildet ist. Die Infusionseinheit hat ein Gehäuse mit einem Fluidreservoir, in dem die Flüssigkeit aufgenommen werden kann, eine Eingabeeinrichtung zur Eingabe von Infusionssteuerbefahlen, eine Ausgabeeinrichtung und eine Kommunikationseinrichtung zum drahtlosen Senden und Empfangen von Signalen an bzw. von der Fernsteuereinheit. Die Fernsteuereinheit hat ein Gehäuse, eine Eingabeeinrichtung zur Eingabe von Infusionssteuerbefehlen, eine Ausgabeeinrichtung und eine Kommunikationseinrichtung zum drahtlosen Senden und Empfangen von Signalen an bzw. von der Infusionseinrichtung Mindestens einer der durch Betätigen einer der Eingabeeinrichtungen erzeugten Infusionssteuerbefehle ist ein verifikationsbedürftiger Befehl, dessen Übermittlung und/oder Ausführung durch ein für den Benutzer wahrnehmbares Verifikationssignal verifiziert wird, welches mittels einer der Ausgabeeinrichtungen ausgegeben wird.

Solche Vorrichtungen werden in der Diabetes-Therapie eingesetzt, um durch Injektion von Insulin den Insulinhaushalt des Patienten auszugleichen. Der Ausgleich des Insulinhaushalts ist wichtig, weil sowohl eine zu hohe Insulingabe, als auch eine zu niedrige Insulingabe für den Patienten überaus schädlich ist.

Im Stand der Technik sind deshalb eine Vielzahl von Geräten bekannt, die entweder als sogenannte "Stand-alone Geräte", also als Einzelgeräte, arbeiten oder zusätzlich eine Fernsteuerung aufweisen, mit der das Infusionsgerät gesteuert werden kann. Die Fernsteuerung hat den Vorteil, dass das Infusionsgerät unter der Kleidung am Körper getragen und auf komfortable Weise über die Fernsteuerung bedient und kontrolliert werden kann. Ein derartiges Gerät ist beispielsweise aus der EP 0 048 423 A2 bekannt

Die WO 01/70307 A1 schlägt eine Infusionseinheit vor, die am Körper getragen wird. Sie weist einen Schacht auf, in den ein "Kommunikationsschiossel" (communication key) eingeschoben werden kann, um die Infusionseinheit mittels einer Fernsteuerung zu steuern. Die Fernsteuerung kann entweder über eine Kabelverbindung oder über eine drahtlose Verbindung mit der Infusionseinheit kommunizieren. Zum Programmieren der Infusionseinheit ist neben einem speziellen Fernsteuergerät auch die Möglichkeit vorgesehen, einen Computer oder einen Handheld-Computer zur Fernsteuerung zu verwenden. Dies ist insbesondere dann bevorzugt, wenn eine aufwendigere Programmierung der Infusionseinheit durch medizinisches Personal stattfinden soll. Zur einfachen Bedienung der Infusionseinheit reicht die spezifische Fernsteuerung aus, mit der lediglich die Bolusrate oder die Basalrate verändert bzw. eingestellt werden kann. Der Kommunikationsschlüssel kann jedoch auch entfernt werden. In diesem Fall arbeitet die Infusionseinheit als Einzelgerät und ist nicht fernsteuerbar.

Auch aus der EP 1109586 B1 ist eine fernsteuerbare Infusionsvorrichtung bekannt. Neben der Infusionseinheit (infusion device) gehört eine Femsteuereinheit (remote commander) zu dem dort beschriebenen System. Mittels des Fernsteuergeräts kann die Infusionseinheit bedient und programmiert werden. Da die Einhaltung der Infusionsmenge für die Gesundheit des Patienten sehr wichtig ist, wird vorgeschlagen, die zur Steuerung der Infusionseinheit übermittelten Infusionssteuerbefehle zu quittieren. Dazu werden an der Infusionseinheit Verfikationssignale in Form einer visuellen oder akustischen Anzeige oder einer Vibration ausgegeben, wenn ein Befehl von der Femsteuereinheit empfangen worden ist. Ein weiteres Verifikationssignal wird dann ausgegeben, wenn der von der Fernsteuereinheit empfangene Befehl ausgeführt wird. Dadurch kann der Benutzer, auch bei Fernsteuerung der Infusionseinheit, die Übertragung der Befehle und die Ausführung der Befehle an der Infusionseinheit durch die Verfikationssignale überwachen. Die Infusionspumpe befindet sich in ständiger Empfangsbereitschaft, um von der Fernsteuereinheit übertragende Signale zu erkennen und zu quittieren.

Es ist Aufgabe der vorliegenden Erfindung, ein Infusionssystem vorzuschlagen, das in seiner Bedienung verbessert ist, um die Bedienung sicherer und fehlertoleranter zu machen. Insbesondere soll dabei der durch die Mensch-Maschine-Schnittstelle gegebene Unsicherheitsfaktor des Gesamtsystems reduziert werden.

Gelöst wird die Aufgabe durch ein Infusionssystem mit den Merkmalen des Patentanspruchs 1.

Das erfindungsgemäße Infusionssystem mit einer Infusionseinheit und einer Femsteuereinheit mit den im Oberbegriff des Patentanspruchs 1 definierten Merkmalen zeichnet sich dadurch aus, dass das Infusionssystem in zwei Betriebsmodi betrieben werden kann und es eine Triggereinrichtung einschließt, durch die zwischen den beiden Betriebsmodi, nämlich einem Fernsteuermodus (engl.; remote control mode) und einem Direktsteuermodus (engl.: direct control mode), umgeschaltet wird. in dem Fernsteuermodus wird mindestens ein verifikationsbedürftiger Infusionssteuerbefehl mittels der Eingabeeinrichtung der Femsteuereinheit (engl.; remote control unit) erzeugt und das entsprechende Verifikationssignal mittels der Ausgabeeinrichtung der Fernsteuereinheit ausgegeben. In dem Direktsteuermodus wird mindestens ein verifikationsbedürftiger Infusionssteuerbefehl mittels der Eingabeeinrichtung der Infusionseinheit (engl.: infusion unit) erzeugt und das entsprechende Verifikationssignal mittels der Ausgabeeinrichtung der Infusionseinheit ausgegeben. Das System arbeitet entweder in dem einen oder in dem anderen Modus.

Als Infusionssteuerbefehie werden dabei diejenigen an einer der Eingabeeinrichtung der Femsteuereinheit oder der Infusionseinheit eingegebenen Befehle bezeichnet, die zur Steuerung der Infusion, beispielsweise zur Einstellung der "Basalrate" oder einer kurzfristigen infusionserhöhung ("Bolus") dienen. Selbstverständlich können auch andere Befehle an den Eingabeeinrichtungen eingegeben werden, beispielsweise um eine Anzeige zu verändern oder um eine interne Uhr einzustellen. Dabei handelt es sich jedoch nicht um Infusionssteuerbefehle im Sinne der Erfindung.

Durch die Erfindung wird eine wesentliche Verbesserung der Bedienungssicherheit erreicht, wobei die auf diesem Gebiet bestehenden besonderen Probleme berücksichtigt werden müssen:
- Diabetes ist eine schwere Krankheit mit hohen, letztlich tödlichen Risiken.
- Etwaige Bedienungsfehler müssen deswegen mit größtmöglicher Sicherheit ausgeschlossen werden.
- Die Benutzer sind infolge der Krankheit und ihres meist höheren Alters in ihrer Bedienungssicherheit eingeschränkt. Die Einschränkungen beziehen sich in vielen Fällen auf das Kurzzeitgedächtnis. Auch die übertragung taktiler Reize ist häufig reduziert.
- Die Bedienung eines für die Diabetes-Therapie geeigneten Infusionssystems muß deshalb möglichst einfach sein. Gefordert wird eine "robuste" Bedienung, in dem Sinne, dass sie durch intuitive Bedienbarkeit Fehler möglichst weitgehend ausschließt.

Im Rahmen der Erfindung wurde festgestellt, dass insoweit eine erhebliche Verbesserung erreicht wird, wenn das Verifikationssignal, mit dem die Eingabe eines Infusionssteuerbefehls quittiert wird, stets (zumindest auch) an derjenigen Einheit des Systems ausgegeben wird, an der die Befehlseingabe erfolgt ist. Obwohl der Wechsel des Ausgabeortes des Verifikationssignals zunächst als Komfortminderung erscheint, die scheinbar zu einer größeren Komplexität und damit schwierigeren Zuordenbarkeit der Verifikationssignale führt, wurde im Rahmen der Untersuchungen, die der Erfindung zugrunde liegen, festgestellt, dass eine wesentliche Erhöhung der Bedienungssicherheit erreicht wird. Durch das "lokal erzeugte" Venfikationssignal erhält der Benutzer eine unmittelbare Bestätigung dafür, dass er an der entsprechenden Einheit (Fembedienungseinheit oder Infusionseinheit) eine Eingabe vorgenommen hat Diese Information ist - informationstheoretisch betrachtet - redundant, weil der Benutzer selbst den Befehl an der entsprechenden Einheit eingegeben hat Es wurde jedoch festgestellt, dass diese Redundanz die Bedienungssicherheit erhöht. Dabei ist zu berücksichtigen, dass ein Infusionssteuerbefehl in der Regel aus einer Sequenz von Einzeibefehlen besteht, die durch das Drücken von Tasten eingegeben werden. Es wurde festgestellt, dass durch die Erfindung die Fähigkeit der Benutzer, diese Sequenz an der jeweils gewählten Eingabeeinrichtung korrekt und vollständig einzugeben, durch das lokal ausgegebene Verifikationssignal deutlich verbessert wird. Beispielsweise wird eine zusätzliche Insulininfusion ("Bolus") durch eine Serie von Tastendrucken programmiert, die jeweils verifiziert werden. Durch das bei Anwendung der Erfindung gegebene lokale Feedback wird intuitiv sichergestellt, dass der Benutzer auch die weiteren Tastendrucke eine Befehlsequenz konsequent (an der selben Eingabeeinheit) vornimmt.

Neben dieser Verbesserung der Bedienung führt die Erfindung in der Regel auch zu einer Einsparung des Energieverbrauchs, weil die in dem jeweiligen Betriebsmodus nicht benötigten Komponenten abgeschaltet werden können. Beispielsweise wird in dem Direktsteuermodus zweckmäßigerweise die Kommunikationseinrichtung der Infusionseinheit abgeschaltet. In dem Fernsteuermodus kann die Ausgabeeinrichtung der Infusionseinheit von der Energieversorgung getrennt werden. Diese Energieeinsparung ist wichtig, weil dadurch die Betriebsdauer bei gegebener Batteriekapazität erhöht wird.

Vorzugsweise ist das System so ausgebildet, dass die Umschaltung zwischen den Betriebsmodi nicht vollautomatisch erfolgt, sondern eine Aktion des Benutzers erfordert, so dass er sich bewußt entscheiden kann (und muß), ob er das Infusionssystem im Fernsteuermodus oder im Direktsteuermodus betreiben möchte. Der Bediener hat also die Freiheit, immer situationsgerecht handeln zu können. Diese Freiheit ist verbunden mit der Sicherheit, dass durch das klare entweder-oder-Prinzip, also entweder Fernsteuermodus oder Direktsteuermodus, sein Handeln intuitiv richtig ist. Unterstützt wird dies vorzugsweise dadurch, dass durch gleiche Bedienungsabläufe sowohl bei der Bedienung über die Femsteuereinheit als auch durch bei der Bedienung der Infusionseinheit im Direktsteuermodus optimal auf die Kundenbedürfnisse reagiert werden kann. Dadurch, dass bei bekannten Systemen die Bedienung parallel sowohl an der Fernsteuereinheit als auch über Tastendrucke an der Infusionseinheit möglich ist, werden die Benutzer häufig verwirrt, was zu Fehleingaben und Fehlbedienungen des Infusionssystems führen kann.

Vorzugsweise ist vorgesehen, dass im Fernsteuermodus alle verifkationsbedürftigen Infusionssteuerbefehle mittels der Eingabeeinrichtung der Fernsteuereinheit erzeugt werden. Dementsprechend werden auch alle daraus resultierenden Verifkationssignale (jedenfalls auch) mittels der Ausgabeeinrichtung der Femsteuereinheit ausgegeben. Dabei werden an der Infusionseinheit vorzugsweise keine Verifikationssignale ausgegeben.

Bevorzugt werden im Direktsteuermodus alle verifikationsbedürftigen Befehle mittels der Eingabeeinrichtung der Infusionseinheit erzeugt und alle entsprechenden Verifikationssignale (jedenfalls auch) mittels der Ausgabeeinheit der Infusionseinheit ausgegeben. Der Benutzer bedient die Infusionspumpe als Stand-alone-Gerät und gibt alle Befehle direkt an der Infusionseinheit ein. Hier werden auch die Verfikationssignale zu den Infusionssteuerbefehlen, die die Infusionspumpe steuern, bevorzugt nur an der Infusionseinheit ausgegeben. Die Femsteuereinheit wird nicht benötigt. Sie kann ausgeschaltet werden. Dies ist beispielsweise vorteilhaft, wenn die Fernsteuereinheit aufgrund eines Defekts ausgefallen ist oder vom Patienten verloren bzw. vergessen wurde. Auch in diesem Falle kann das erfindungsgemäße Infusionssystem weiterhin in der gewohnten Weise bedient werden, so dass die von dem System ausgeführten lebenserhaltenden Funktionen sicher durchgeführt werden können.

Die Verifikationssignale können als optische Signale (beispielsweise mittels einer oder mehrerer Kontrollleuchten oder durch Anzeige von Klartext auf einem Display) als akustische Signale (z.B. in Form von Tönen oder als Sprachausgabe) oder als fühlbare Signale (z.B. Vibrationen) ausgegeben werden. Auch eine Kombination dieser Signalformen ist möglich. Wenn in einem System unterschiedliche Signaltypen verwendet werden, kann die Ausgabe von einer vom Benutzer festgelegten Einstellung abhängen oder vom Ort der Ausgabe, also davon, ob das Verifikationssignal an der Infusionseinheit oder an der Fernsteuereinheit ausgegeben wird.

Das für den Benutzer wahrnehmbare Verifikationssignal ist bevorzugt ein "Befehlverifikationssignal", welches angibt, dass ein Infusionssteuerbefehl von der Infusionseinheit empfangen worden ist.

Gemäß einer anderen bevorzugten Ausführungsform ist das für den Benutzer wahrnehmbare Verifikationssignal ein "Ausführungsverifikationssignal", das anzeigt, wenn ein Injektionssteuerungsbefehl von der Infusionseinheit ausgeführt wird. Dabei kann weiter unterschieden werden, ob gerade mit der Ausführung des Injektionssteuerungsbefehls begonnen wurde oder ob die Ausführung beendet worden ist. Beides kann in Form eines Ausführungsverifikationssignals angegeben werden.

Es ist ebenfalls möglich, mehrere Verifikationssignale hintereinander auszugeben. Wird nach der Ausgabe eines Befehlsverifikationssignals ein Ausführungsverifikationssignal ausgegeben, so spricht man von einer "Double-Indication". Werden nach dem Befehisverifikationssignal zwei Ausführungsverifikationssignale erzeugt, einmal zur Anzeige, dass mit der Ausführung des Infusionssteuerbefehls begonnen wurde, und einmal zur Anzeige, dass die Ausführung des Infusionssteuerbefehls abgeschlossen wurde, so wird von einer "Triple-Indication" gesprochen.

Im Femsteuermodus wird bevorzugt eine Double-Indication an der Fernsteuereinheit ausgegeben, wenn ein verifikationsbedürftiger Befehl mittels der Eingabeeinrichtung der Femsteuereinheit erzeugt wurde. Optional kann an der Infusionseinheit ein zusätzliches Verifikationssignal ausgegeben werden. Im Direktsteuermodus ist das gleiche Prinzip durchführbar. Auch hier wird vorzugsweise eine Double-Indication ausgegeben. Zusätzlich kann optional an der Fernsteueteinheit ein Verifikationssignal ausgegeben werden. Die unterschiedlichen Ausgabemöglichkeiten können vom Hersteller direkt in dem Infusionssystem implementiert sein; sie können aber auch benutzerabhängig ausgewählt bzw. festgelegt sein. Damit ist neben der situativen Umschaltung der Betriebsmodi durch den Benutzer auch eine situative Festlegung der Ausgabe der Verifikationssignale möglich.

Bevorzugte Ausführungsbeispiele werden in den nachfolgenden Zeichnungen beschrieben. Die darin dargestellten technischen Merkmale können einzeln oder in Kombination verwendet werden, um bevorzugte Ausgestaltungen der Erfindung zu schaffen. Es zeigen:
- Fig. 1: ein Prinzipbild eines Infusionssystems mit einer Infusionseinheit und einer Fernsteuereinheit;
- Fig. 2: ein detailliertes Blockdiagramm der Infusionseinheit und der Fernsteuereinheit aus Figur 1;
- Fig. 3: ein Blockdiagramm einer alternativen Fernsteuereinheit;
- Fig. 4: ein Blockdiagramm zur Erläuterung der Funktion einer Triggereinrichtung;
- Fig. 5: eine Prinzipskizze einer alternativen Ausführungsform des Infusionssystems mit Infusionseinheit und Fernsteuereinheit.

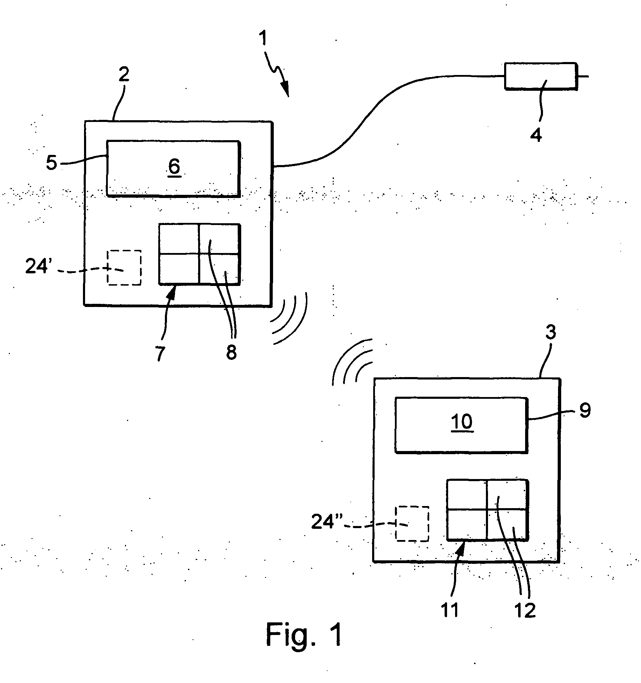

Figur 1 zeigt ein Infusionssystem 1 mit einer Infusionseinheit 2 und einer Femsteuereinheit 3. Die Infusionseinheit 2 pumpt eine Flüssigkeit, insbesondere Insulin, durch einen dünnen Schlauch zu einer Injektionseinheit 4 mit einer Nadel, die am Körper des Patienten befestigt wird. Wie in der schematischen Darstellung gezeigt ist, hat die Infusionseinheit 2 eine Ausgabeeinrichtung 5 (output device), die als Display 6 ausgeführt ist. Auf dem Display können in visueller Form Verifikationssignale angezeigt werden, um Infusionssteuerbefehle (injection control commands) zur Steuerung der Infusionseinheit 2 zu quittieren.

Eine Eingabeeinrichtung 7 (input device) hat Tasten 8, mit deren Hilfe die Infusionseinheit 2 gesteuert wird. Über die Tasten 8 kann sowohl die Basalrate der Infusionseinheit 2 verändert, als auch ein Bolus eingestellt werden. Mittels der Tasten 8 kann auch die komplette Funktionssteuerung der Infusionseinheit 2 erfolgen.

Die Femsteuereinheit 3 hat ebenfalls eine Ausgabeeinrichtung 9, die als Display 10 zur visuellen Anzeige ausgeführt ist Auf dem Display 10 werden neben den als Antwort auf die Infusionssteuerbefehle erzeugten Verifikationssignalen auch weitere Anzeigen ausgegeben. Beispielsweise können zusätzlich die Uhrzeit oder andere relevante Daten dargestellt werden.

Eine Eingabeeinrichtung 11 der Femsteuereinheit 3 entspricht im wesentlichen der Eingabeeinrichtung 7 der Infusionseinheit 2. Die Eingabeeinrichtung 11 ist mit Tasten 12 ausgestaltet. Durch eine ähnliche Gestaltung der Eingabeeinrichtungen 7,11 und der Ausgabeeinrichtungen 5,9 ist für den Benutzer die Bedienung sowohl an der Fernsteuereinheit 3 als auch an der Infusionseinheit 2 identisch, so dass es für den Patienten keinen Unterschied macht, ob er das Infusionssystem 1 über die Infusionseinheit 2 oder die Fernsteuereinheit 3 bedient. Auf diese Weise werden Bedienfehler minimiert und der Bedienkomfort erhöht.

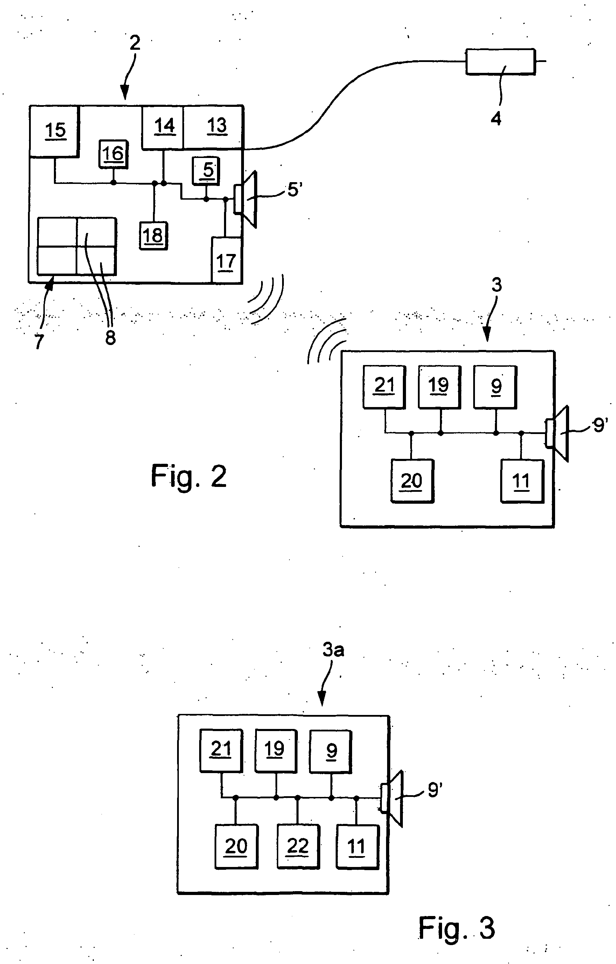

Figur 2 zeigt ein Blockdiagramm der Infusionseinheit 2 und der Femsteuereinheit 3. Die Infusionseinheit 2 weist ein Fluidreservoir 13 auf, in dem die Flüssigkeit aufgenommen wird, die über die Injektionseinheit 4 in den Körper des Patienten appliziert wird. An das Fluidreservoir 13 ist eine Pumpe 14 angeschlossen, durch deren Steuerung die zu applizierende Infusionsmenge geregelt wird. Insbesondere wird durch die Geschwindigkeit der Pumpenförderung die in der Infusionseinheit 2 eingestellte Basalrate festgelegt. Ein wahlweise zusätzlich zu applizierender Bolus läßt sich durch die Dauer und das Ausmaß einer zeitweisen Erhöhung der Pumpgeschwindigkeit steuern.

Die Infusionseinheit 2 umfaßt ferner eine Stromversorgungseinheit (Power Supply Unit) 15, einen Mikroprozessor 16 und eine Kommunikationseinrichtung (communication device) 17 zum drahtlosen Senden und Empfangen von Signalen an bzw. von der Fernsteuereinheit 3. Die Stromversorgungseinheit 15 versorgt alle Komponenten der lnfusionseinheit 2 mit Energie. Die Ausgabeeinrichtung 5 ist als Block dargestellt. Ein Lautsprecher 5', der ebenfalls zur Ausgabeeinrichtung 5 gehört, dient zur akustischen Ausgabe von Verifikationssignaleri an der Infusionseinheit 2. Eine Triggereinrichtung 18 dient zum Umschalten zwischen dem Fernsteuermodus und dem Direktsteuermodus des Infusionssystems 1.

Die Femsteuereinheit 3 umfaßt neben der Eingabeeinrichtung 11 und der Ausgabeeinrichtung 9 einen Lautsprecher 9' zur akustischen Ausgabe von Verifikationssignalen. Ein Mikroprozessor 19 verarbeitet die Eingaben der Eingabeeinrichtung 11 und die von der Infusionseinheit 2 drahtlos übertragenen Signale, die von einer Kommunikationseinrichtung 20 empfangen werden. Die Kommunikationseinrichtung 20 arbeitet bidirektional. Sie sendet auch Signale von der Femsteuereinheit 3 an die Infusionseinheit 2. Eine Stromversorgungseinheit 21 versorgt die Femsteuereinheit 3 und alle Komponenten mit der notwendigen Energie.

Die Kommunikationseinrichtungen 17 und 20 sind bevorzugt als sogenannte Transceiver ausgeführt; sie umfassen also einen Transmitter zum Senden von Signalen wie auch einen Receiver, um Signale zu empfangen. Die übertragenen Signale sind vorzugsweise elektromagnetische Wellen. Alternativ kann die Kommunikation zwischen den Kommunikationseinrichtungen 17 und 20 über optische Signale (insbesondere über Infrarot-Signale) erfolgen. Die Signale können auf übliche Weise codiert sein.

Figur 3 zeigt eine alternative Ausführungsform der Fernsteuereinheit 3, die neben den bereits beschriebenen Komponenten auch eine Triggereinrichtung 22 umfaßt. In der Infusionseinheit 2 muß dann keine Triggereinrichtung 18 vorgesehen sein.

In einer bevorzugten Ausführungsform des Infusionssystems 1 wird beim Umschalten in den Direktsteuermodus die Kommunikationseinrichtung 17 der Infusionseinheit 2 deaktiviert. Sie wird im Direktsteuermodus nicht benötigt, weil keine Signale an die Fernsteuereinheit 3 übertragen werden müssen.

Besonders bevorzugt wird die Kommunikatiönseindchtung 17 der Infusionseinheit 2 von dem Prozessor 16 getrennt, wenn sie deaktiviert ist. Der Prozessor 16 sendet dann keine Signale an die Kommunikationseinrichtung 17. Zusätzlich oder alternativ kann die Kommunikationseinrichtung 17 von der Stromversorgungseinheit 15 getrennt werden. Die Deaktivierung der Kommunikationseinrichtung 17 ist aus mehreren Gründen vorteilhaft. Zum einen wird Energie eingespart, so dass die in der Stromversorgungseinheit 15 gespeicherte Energie länger ausreicht, um die Infusionseinheit 2 zu betreiben. Gleichzeitig werden störende Einflüsse auf das System und die Umgebung reduziert. Das Ausschalten der Kommunikationseinrichtung 17 kann beispielsweise durch einfache interrupt-gesteuerte Routinen in dem Mikroprozessor 16 ausgeführt werden, bei der alle Tasten 8 überwacht werden. Dazu kann ein einfacher Schalttransistor pro zuschaltender Leitung verwendet werden.

Bevorzugt wird beim Umschalten in den Femsteuermodus die Ausgabeeinrichtung 5 der Infusionseinheit 2 deaktiviert. Da im Femsteuermodus alle Verifikationssignale an der Ausgabeeinrichtung 9 der Femsteuereinheit 3 bzw. 3a ausgegeben werden, muß die Ausgabeeinrichtung 5 nicht aktiv sein. Die Ausgabeeinrichtung 5 kann deshalb abgeschaltet werden. Dazu veranlaßt der Mikroprozessor 16, dass eine Trennung von der Stromversorgungseinheit 15 stattfindet. Die Ausgabeeinrichtung 5 wird erst dann wieder aktiviert, also mit Strom versorgt, wenn die Triggereinrichtung 18 bzw. 22 das Infusionssystem 1 in den Direktsteuermodus umschaltet.

Die Deaktivierung der Ausgabeeinrichtung 5 kann auch dadurch stattfinden, dass der Mikroprozessor 16 keine Signale an die Ausgabeeinrichtung 5 ausgibt. Zusätzlich kann auch der Lautsprecher 5' deaktiviert werden. Alternativ ist es jedoch möglich, dass der Lautsprecher 5' aktiv bleibt, so dass zusätzlich Verifikationssignale an der Infusionseinheit 2 in Form von akustischen Signalen ausgegeben werden. Darüber hinaus kann trotz Abschalten der Ausgabeeinrichtung 5 ein Wamton am Lautsprecher 5' ausgegeben werden, falls beispielsweise die Flüssigkeitsmenge im Fluidreservoir 13 einen kritischen Wert unterschreitet oder die von der Stramversorgungseinheit 15 abgegebene Spannung zu gering wird.

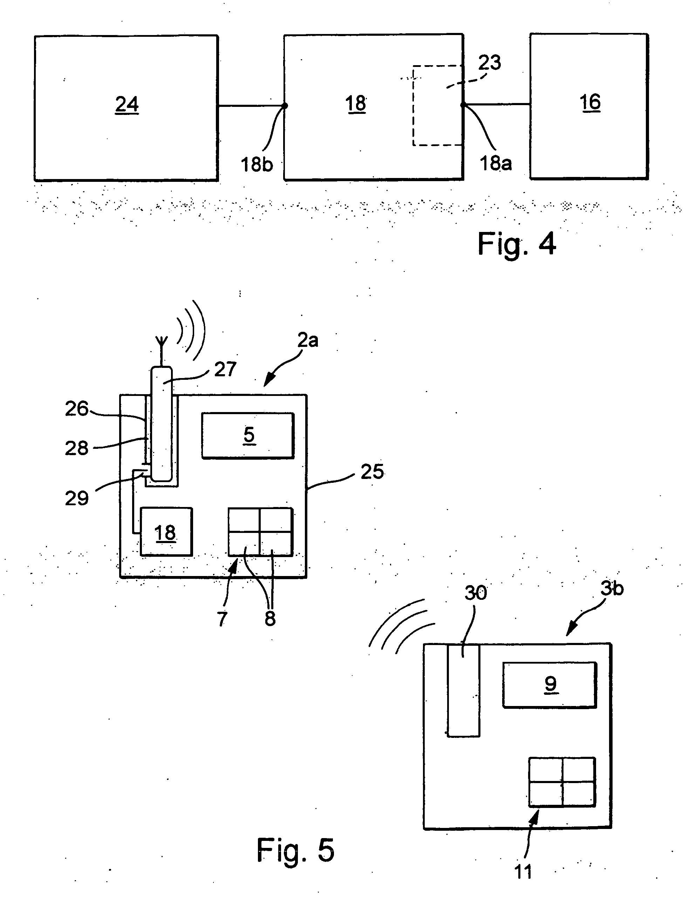

Anhand von Figur 4 läßt sich die Funktion der Triggereinrichtung 18 erläutern. Um die Umschaltung zwischen dem Fernsteuermodus und dem Direktsteuermodus zu bewirken, erzeugt sie an ihrem Ausgang 18a ein Steuersignal für das Infusionssystem, das mittels bekannter elektronischer Mittel die Umschaltung zwischen den Betriebszuständen bewirkt. In der Regel bildet das Steuersignal einen Befehl für den Mikroprozessor 16, der seinerseits dann die Betriebsmodi-Umschaltung bewirkt.

Die Triggereinrichtung 18 wird ihrerseits durch ein Triggersignal gesteuert, das an ihren Eingang 18b übermittelt wird. Die Mittel, mit denen das Triggersignal erzeugt wird, reagieren dabei in irgendeiner Weise auf Handlungen des Benutzers oder Veränderungen in der Umgebung des Infusionssystems. Sie werden deshalb allgemein als Triggersensor 24 bezeichnet. Unterschiedliche Ausgestaltungen der Erfindung unterscheiden sich durch die Art der Erzeugung des Triggersignals oder mit anderen Worten durch unterschiedliche Ausgestaltungen des Triggersensors 24.
a) Im einfachsten Fall dient ein manuell betätigbarer, speziell für diesen Zweck vorgesehener Eingabeknopf (dedicated input key) als Sensor zur Erzeugung des Triggersignals. Ein solcher Eingabeknopf kann sowohl an der Infusionseinheit 2, als auch an der Fernsteuereinheit 3 vorgesehen sein (vgl. Figur 1 und 2; optionale Eingabeknöpfe 24' bzw. 24"). Selbstverständlich sind zahlreiche Varianten möglich, beispielsweise eine druckempfindliche Fläche in einem als "touch screen" ausgebildeten Display 6,10 (Figur 1).
b) Der Sensor 24 kann aus einer Kombination der ohnehin vorhandenen Tasten 8,12 der Eingabeeinrichtungen 7,11 mit einer Logikschaltung bestehen, durch die eine Kombination von Tastenbetätigungen (beispielsweise das gleichzeitige Drücken von zwei bestimmten Tasten) als manuell erzeugter Befehl zum Umschalten der Betriebsmodi interpretiert und demzufolge ein Triggersignal generiert und an den Eingang 18a derTriggereinrichtung 18 übermittelt wird.
c) Der Sensor 24 kann von einer Eingabewechsel-Erkennungselektronik (input change detection electronics) gebildet werden, die detektiert, wenn der Benutzer bei der Bedienung von der Infusionseinheit 2 zu der Fernsteuereinheit 3 oder umgekehrt wechselt. Wenn sich also das System beispielsweise in dem Direktsteuermodus befindet und der Benutzer einen Infusionssteuerbefehl an der Fernsteuereinheit 3 eingibt, wird dies von der Eingabewechsel-Erkennungselektronik erkannt und ein Triggersignal an die Triggereinrichtung 18 übermittelt. Nun werden alle Verifikationssignale, die zur Quittierung eines Infusionssteuerbefehls dienen, an der Femsteuereinheit ausgegeben. Auch eine solche Eingabewechsel-Erkennungselektronik bildet demzufolge einen Sensor 24, der ein Triggersignal für die Triggereinrichtung 18 erzeugt.
d) Schließlich kann der Sensor 24 auch von einer Fernsteuereinheit-Funktionserkennungselektronik (remote control function detection electronics) gebildet werden. Diese erkennt, wenn die Voraussetzungen für die Kommunikation zwischen einer Fernsteuereinheit 3 und einer Infusionseinheit 2 (beide Geräte eingeschaltet, Kommunikationseinrichtungen beider Einheiten in Betrieb, sichere Datenübertragung sichergestellt) vorliegen. Unter diesen Voraussetzungen erzeugt eine solche Femsteuereinheit-FunktionSerkennungselektronik ein Triggersignal, dessen Übermittlung an die Triggereinrichtung 18 die Einschaltung des Fernsteuermodus bewirkt.

In den Fällen a) und b) ist das Triggersignal ein manuell erzeugtes Signal.

Der Fall c) ist ein Beispiel dafür, dass das Triggersignal infolge der Eingabe eines beliebigen Befehls an einer der Eingabeeinrichtungen 7,11, bevorzugt infolge der Eingabe eines Infusionssteuerbefehts, erzeugt werden kann. Ein derartiges Triggersignal wird auch als semiautomatisch erzeugtes Signal bezeichnet.

Der Fall d) zeigt, dass das Triggersignal auch durch Erkennen eines Signalaustauschs zwischen den Kommunikationseinrichtungen 17,20 der Fernsteuereinheit 3,3a,3b und der Infusionseinheit 2,2a erzeugt werden kann. Ein derartiges Triggersignal wird auch als automatisch erzeugtes Signal bezeichnet.

Bei jeder der beschriebenen Ausführungsformen kann vorgesehen sein, dass nur die Umschaltung von einem Standardmodus (beispielsweise dem Direktsteuermodus) in den anderen Modus (Fernsteuermodus) durch ein spezielles Triggersignal ausgelöst wird, während die Rückkehr in den Standardmodus durch ein (optionales) zeitabhängiges Steuerglied 23 ausgelöst wird, dass in Figur 4 gestrichelt dargestellt ist. Das Zeitintervall, nach dem das zeitabhängige Steuerglied 23 das Umschalten in den Standardmodus bewirkt, kann seitens des Herstellers in dem System fest einprogrammiert oder durch den Benutzer veränderbar sein.

Die Mittel zur Realisierung der erläuterten Konfigurationen des Sensors 24 können elektronisch sehr unterschiedlich gestaltet sein und sind bekannt. Insbesondere ist es möglich, dass die anhand der Figur 4 in Form von gesonderten elektronischen Bauteilen (Sensor 24, Triggereinrichtung 18 und gegebenenfalls zeitabhängiges Steuerglied 23) erläuterten Funktionen teilweise oder vollständig durch Software realisiert werden. Die vorstehenden Erläuterungen gelten selbstverständlich in gleicher Weise, wenn sich die Triggereinrichtung in der Femsteuereinheit befindet.

Figur 5 stellt eine weitere Ausführungsform eines Infusionssystems 1 dar, das eine Infusionseinheit 2a und eine Femsteuereinheit 3b umfaßt. In dem Gehäuse 25 der Infusionseinheit 2a ist eine Aussparung 26 vorgesehen, in die eine als Transceiver-Modul 27 ausgebildete Kommunikationseinfichtung eingesetzt wird. Die Aussparung 26 bildet einen Aufnahmeraum 28, der passend zu dem Transceiver-Modul 27 ausgebildet ist. Im unteren Bereich des Aufnahmeraums 28 ist ein Kontakt 29 angeordnet, der dazu dient, das Vorhandensein bzw. Fehlen des Transceivers zu erkennen. Sobald das herausnehmbare Transceiver-Modul 27 in die Infusionseinheit 2a eingesteckt wird, wird der Kontakt 29 geschlossen. Hier bildet folglich der Kontakt 29 einen Sensor, der ein Triggersignal erzeugt, dass am Eingang der Triggereinrichtung 18 anliegt, so dass der Betriebsmodus in den Fernsteuermodus umgeschaltet wird.

Die Entnahme des Transceiver-Moduls 27 aus dem Aufnahmeraum 28 öffnet den Kontakt 29, so dass die Triggereinrichtung 18 in den Direktsteuermodus umschaltet. Eine Kommunikation mit der Femsteuereinheit 3b ist dann nicht mehr möglich. Alle Eingaben werden an der Eingabeeinrichtung 7 der Infusionseinheit 2a erwartet. Durch das Ein- bzw. Ausstecken des Transceiver-Moduls 27 wird das Triggersignal folglich manuell erzeugt.

Während des Direktsteuermodus des Infusionssystems 1 kann das Transceiver-Modul 27 in einer entsprechenden Ausnehmung 30 in der Femsteuereinheit 3b aufbewahrt werden. Auf diese Weise kann es nicht verloren gehen. Erst wenn ein Umschalten in den Fernsteuermodus erfolgen soll, wenn der Patient also sein Infusionssystem 1 über die Femsteuereinheit 3b bedienen möchte, wird das Transceiver-Modul 27 benötigt. Der Patient hat dann sowohl die Femsteuereinheit 3b als auch das Transceiver-Modul 27 direkt zusammen bei der Hand. Er muß es nur noch von der Femsteuereinheit 3b in die Infusionseinheit 2a umstecken.

Anstelle des Transceiver-Moduls 27 kann auch ein herausnehmbares Antennenmodul vorgesehen sein. Das Antennenmodul wird dann auf die gleiche Weise verwendet wie das Transceiver-Modul 27.

## Patentansprüche

1. Infusionssystem mit einer Infusionseinheit und einer Femsteuereinheit,
wobei die Infusionseinheit zur Anordnung außerhalb des Körpers und zur Infusion einer Flüssigkeit in den Körper ausgebildet ist und umfaßt:
- ein Gehäuse mit einem Fluidreservoir für die Flüssigkeit,
- eine Eingabeeinrichtung zur Eingabe von Infusionssteuerbefehlen
- eine Ausgabeeinrichtung
- eine Kommunikationseinrichtung zum drahtlosen Senden und Empfangen von Signalen an bzw. von der Fernsteuereinheit;
wobei die Fernsteuereinheit umfaßt:
- ein Gehäuse,
- eine Eingabeeinrichtung zur Eingabe von Infusionssteuerbefehlen,
- eine Ausgabeeinrichtung,
- eine Kommunikationseinrichtung zum drahtlosen Senden und Empfangen von Signalen an bzw. von der Infusionseinheit, und
wobei mindestens ein durch Betätigen einer der Eingabeeinrichtungen erzeugter lnfusionssteuerbefehl ein venfikationsbedürftiger Befehl ist, dessen Übermittlung und/oder Ausführung durch ein für den Benutzer wahrnehmbares Verifikationssignal, welches mittels einer der Ausgabeeinrichtungen ausgegeben wird, verifiziert wird,
**dadurch gekennzeichnet, dass**
das Infusionssystem (1)
in zwei unterschiedlichen Betriebsmodi betrieben werden kann, nämlich
a) einem Femsteuermodus, in dem mindestens ein verifikationsbedürftiger Befehl mittels der Eingabeeinrichtung (11) der Fernsteuereinheit (3,3a,3b) erzeugt und das entsprechende Verifikationssignal mittels der Ausgabeeinrichtung (9) der Fernsteuereinheit (3,3a,3b) ausgegeben wird und
b) einem Direktsteuermodus, in dem mindestens ein verifikationsbedürftiger Befehl mittels der Eingabeeinrichtung (7) der Infusionseinheit (2,2a) erzeugt und das entsprechende Verifikationssignal mittels der Ausgabeeinrichtung (5) der Infusionseinheit (2,2a) ausgegeben wird,
und eine Triggereinrichtung (18,22) einschließt, durch die zwischen dem Fernsteuermodus und dem Direktsteuermodus umgeschaltet wird.

2. Infusionssystem nach Anspruch 1, **dadurch gekennzeichnet, dass** in dem Fernsteuermodus alle verifikationsbedürftigen Befehle mittels der Eingabeeinrichtung (11) der Fernsteuereinheit (3,3a,3b) erzeugt und alle entsprechenden Verifikationssignale mittels der Ausgabeeinrichtung (9) der Fernsteuereinheit (3,3a,3b) ausgegeben werden.

3. Infusionssystem nach Anspruch 1, **dadurch gekennzeichnet, dass** in dem Direktsteuermodus alle verifikationsbedürftigen Befehle mittels der Eingabeeinrichtung (7) der Infusionseinheit (2,2a) erzeugt und alle entsprechenden Verifikationssignale mittels der Ausgabeeinrichtung (5) der Infusionseinheit (2,2a) ausgegeben werden.

4. Infusionssystem nach einem der Ansprüche 1 bis 3, **dadurch gekennzeichnet, dass** die Triggereinrichtung (18,22) zwischen den Betriebsmodi umschaltet, wenn an ihren Eingang ein Triggersignal übertragen wird.

5. lnfusionssystem nach Anspruch 4, **dadurch gekennzeichnet, dass** das Triggersignal ein manuell erzeugtes Signal ist.

6. Infusionssystem nach Anspruch 4, **dadurch gekennzeichnet, dass** das Triggersignal infolge der Eingabe eines beliebigen Befehls an einer der Eingabeeinrichtungen (7,11), besonders bevorzugt infolge der Eingabe eines lnfusionssteuerbefehts, erzeugt wird.

7. Infusionssystem nach Anspruch 4, **dadurch gekennzeichnet dass** das Triggersignal durch Erkennen eines Signalaustauschs zwischen den Kommunikationseinrichtungen (17,20) der Fernsteuereinheit (3,3a,3b) und der Infusionseinheit (2,2a) erzeugt wird.

8. Infusionssystem nach einem der vorhergehenden Ansprüche, **dadurch gekennzeichnet, dass** beim Umschalten in den Direktsteuermodus die Kommunikationseinrichtung (17) der Infusionseinheit (2,2a) deaktiviert wird.

9. Infusionssystem nach Anspruch 8, **dadurch gekennzeichnet, dass** die Kommunikationseinrichtung (17) der Infusionseinheit (2,2a) von einem in der Infusionseinheit (2,2a) angeordneten Prozessor (16) und/oder einer Stromversorgungseinheit (15) der Infusionseinheit (2,2a) getrennt wird, wenn die Kommunikationseinrichtung (17) deaktiviert ist.

10. Infusionssystem nach einem der vorhergehenden Ansprüche, **dadurch gekennzeichnet, dass** beim Umschalten in den Fernsteuermodus die Ausgabeeinrichtung (5) der Infusionseinheit (2,2a) deaktiviert wird.

11. Infusionssystem nach einem der vorhergehenden Ansprüche, **dadurch gekennzeichnet, dass** das für den Benutzer wahrnehmbare Verifikafionssignal ein Befehlsverifikationssignal ist, durch das angezeigt wird, dass ein lnfusionssteuerbefehl von der Infusionseinheit (2,2a) empfangen worden ist.

12. Infusionssystem nach einem der vorhergehenden Ansprüche, **dadurch gekennzeichnet, dass** das für den Benutzer wahrnehmbare Verifikationssignal ein Ausführungsverifikafionssignal ist, durch das angezeigt wird, wenn ein Infusionssteuerbefehl von der Infusionseinheit (2,2a) ausgeführt wird.

13. Infusionssystem nach einem der vorhergehenden Ansprüche, **dadurch gekennzeichnet, dass** die Triggereinrichtung (18,22) ein zeitabhängiges Steuerglied (23) umfaßt, so dass nach Umschalten in den Direktsteuermodus nach einer vorbestimmten Zeit automatisch in den Fernsteuermodus zurückgeschaltet wird.

## Claims

1. An infusion system having an infusion unit and a remote control unit, the infusion unit being adapted to be located outside the body and to infuse a liquid into the body and comprising:
- a housing with a fluid reservoir for the liquid,
- an input device for inputting infusion control commands,
- an output device,
- a communication device for wirelessly transmitting signals to and receiving signals from the remote control unit;
the remote control unit comprising:
- a housing,
- an input device for inputting infusion control commands
- an output device,
- a communication device for wirelessly transmitting signals to and receiving signals from the infusion unit, and
wherein at least one infusion control command generated by actuating one of the input devices is a command requiring verification, and transmission and/or execution of said command is verified by a verification signal perceivable to the user, said verification signal being outputted by one of the output devices,
**characterized in that**
the infusion system (1)
is adapted for operating in two different operating modes, namely
a) a remote control mode, in which at least one command requiring verification is generated by the input device (11) of the remote control unit (3, 3a, 3b) and the corresponding verification signal is output by the output device (9) of the remote control unit (3, 3a, 3b) and
b) a direct control mode, in which at least one command requiring verification is generated by the input device (7) of the infusion unit (2, 2a) and the corresponding verification signal is outputted by the output device (5) of the infusion unit (2, 2a),
and includes a trigger device (18, 22) for switching between the remote control mode and the direct control mode.

2. The infusion system according to Claim 1, **characterized in that**, in the remote control mode, all commands requiring verification are generated by the input device (11) of the remote control unit (3, 3a, 3b) and all corresponding verification signals are outputted by the output device (9) of the remote control unit (3, 3a, 3b).

3. The infusion system according to Claim 1, **characterized in that**, in the direct control mode, all commands requiring verification are generated by the input device (7) of the infusion unit (2, 2a) and all corresponding verification signals are outputted by the output device (5) of the infusion unit (2, 2a).

4. The infusion system according to anyone of Claims 1 to 3, **characterized in that** the trigger device (18, 22) switches between the operating modes when a trigger signal is transmitted to its input.

5. The infusion system according to Claim 4, **characterized in that** the trigger signal is a manually generated signal.

6. The infusion system according to Claim 4, **characterized in that** the trigger signal is generated as a result of the input of any arbitrary command at one of the input devices (7, 11), especially preferably as a result of the input of an infusion control command.

7. The infusion system according to Claim 4, **characterized in that** the trigger signal is generated by detecting a signal exchange between the communication devices (17, 20) of the remote control unit (3, 3a, 3b) and the infusion unit (2, 2a).

8. The infusion system according to anyone of the preceding claims, **characterized in that** the communication device (17) of the infusion unit (2, 2a) is deactivated upon the switching into the direct control mode.

9. The infusion system according to Claim 8, **characterized in that** the communication device (17) of the infusion unit (2, 2a) is disconnected from a processor (16) of the infusion unit (2, 2a) and/or a power supply unit (15) of the infusion unit (2, 2a) when the communication device (17) is deactivated.

10. The infusion system according to anyone of the preceding claims, **characterized in that** the output device (5) of the infusion unit (2, 2a) is deactivated upon the switching into the remote control mode.

11. The infusion system according to anyone of the preceding claims, **characterized in that** the verification signal perceivable to the user is a command verification signal, indicating that an infusion control command has been received by the infusion unit (2, 2a).

12. The infusion system according to anyone of the preceding claims, **characterized in that** the verification signal perceivable to the user is an execution verification signal, which indicates when an infusion control command has been executed by the infusion unit (2, 2a).

13. The infusion system according to one of the preceding claims, **characterized in that** the trigger device (18, 22) comprises a time-dependent control element (23), so that after switching into the direct control mode, the system is switched back automatically into the remote control mode after a predetermined time.

## Revendications

1. Système de perfusion, comportant une unité de perfusion et une unité de télécommande,
l'unité de perfusion étant conçue pour être agencée en dehors du corps et pour administrer un liquide dans le corps, et comportant :
■ un boîtier avec un réservoir pour le liquide,
■ un dispositif d'entrée pour entrer les instructions de commande de la perfusion,
■ un dispositif de sortie,
■ un dispositif de communication pour l'émission sans fil de signaux vers l'unité de télécommande et la réception sans fil de signaux délivrés par cette dernière ;
l'unité de télécommande comportant :
■ un boîtier,
■ un dispositif d'entrée pour entrer les instructions de commande de la perfusion,
■ un dispositif de sortie,
■ un dispositif de communication pour l'émission sans fil de signaux vers l'unité de perfusion et la réception sans fil de signaux délivrés par cette dernière, et
au moins une instruction de commande de la perfusion, générée par l'actionnement de l'un des dispositifs d'entrée, étant une instruction nécessitant une vérification, dont la transmission et/ou l'exécution est vérifiée par un signal de vérification, qui est perceptible par l'utilisateur et qui est édité par l'un des dispositifs de sortie,
**caractérisé en ce que**
le système de perfusion (1)
peut être utilisé dans deux modes de service différents, à savoir
a) un mode de commande à distance, dans lequel au moins une instruction nécessitant une vérification est générée au moyen du dispositif d'entrée (11) de l'unité de télécommande (3, 3a, 3b) et le signal de vérification correspondant est édité par le dispositif de sortie (9) de l'unité de télécommande (3, 3a, 3b), et
b) un mode de commande direct, dans lequel au moins une instruction nécessitant une vérification est générée au moyen du dispositif d'entrée (7) de l'unité de perfusion (2, 2a) et le signal de vérification correspondant est édité par le dispositif de sortie (5) de l'unité de perfusion (2, 2a),
et comporte un dispositif de déclenchement (18, 22) qui permet de commuter entre le mode de commande à distance et le mode de commande direct.

2. Système de perfusion selon la revendication 1, **caractérisé en ce que**, dans le mode de commande à distance, toutes les instructions nécessitant une vérification, générées par le dispositif d'entrée (11) de l'unité de télécommande (3, 3a, 3b), et tous les signaux de vérification correspondants sont édités par le dispositif de sortie (9) de l'unité de télécommande (3, 3a, 3b).

3. Système de perfusion selon la revendication 1, **caractérisé en ce que**, dans le mode de commande direct, toutes les instructions nécessitant une vérification, générées par le dispositif d'entrée (7) de l'unité de perfusion (2, 2a), et tous les signaux de vérification correspondants sont édités par le dispositif de sortie (5) de l'unité de perfusion (2, 2a).

4. Système de perfusion selon l'une quelconque des revendications 1 à 3, **caractérisé en ce que** le dispositif de déclenchement (18, 22) commute d'un mode de service à l'autre lorsqu'un signal de déclenchement est transmis à son entrée.

5. Système de perfusion selon la revendication 4, **caractérisé en ce que** le signal de déclenchement est un signal généré manuellement.

6. Système de perfusion selon la revendication 4, **caractérisé en ce que** le signal de déclenchement est généré à la suite d'une instruction quelconque sur l'un des dispositifs d'entrée (7, 11), de manière particulièrement préférée à la suite d'une instruction de commande de la perfusion.

7. Système de perfusion selon la revendication 4, **caractérisé en ce que** le signal de déclenchement est généré par la détection d'un échange de signaux entre les dispositifs de communication (17, 20) de l'unité de télécommande (3, 3a, 3b) et de l'unité de perfusion (2, 2a).

8. Système de perfusion selon l'une quelconque des revendications précédentes, **caractérisé en ce que**, lors de la commutation dans le mode de commande direct, le dispositif de communication (17) de l'unité de perfusion (2, 2a) est désactivé.

9. Système de perfusion selon la revendication 8, **caractérisé en ce que** le dispositif de communication (17) de l'unité de perfusion (2, 2a) est séparé d'un processeur (16), monté dans l'unité de perfusion (2, 2a), et/ou d'une unité d'alimentation électrique (15) de l'unité de perfusion (2, 2a), lorsque le dispositif de communication (17) est désactivé.

10. Système de perfusion selon l'une quelconque des revendications précédentes, **caractérisé en ce que**, lors de la commutation dans le mode de commande à distance, le dispositif de sortie (5) de l'unité de perfusion (2, 2a) est désactivé.

11. Système de perfusion selon l'une quelconque des revendications précédentes, **caractérisé en ce que** le signal de vérification, qui est perceptible par l'utilisateur, est un signal de vérification d'instruction, qui indique qu'une instruction de commande de la perfusion a été reçue par l'unité de perfusion (2, 2a).

12. Système de perfusion selon l'une quelconque des revendications précédentes, **caractérisé en ce que** le signal de vérification, qui est perceptible par l'utilisateur, est un signal de vérification d'exécution, qui indique qu'une instruction de commande de la perfusion a été exécutée par l'unité de perfusion (2, 2a).

13. Système de perfusion selon l'une quelconque des revendications précédentes, **caractérisé en ce que** le dispositif de déclenchement (18, 22) comporte un organe de commande (23) asservi au temps, de telle sorte qu'après une commutation dans le mode de commande direct, il commute automatiquement après une durée prédéfinie dans le mode de commande à distance.
